# EUROPEAN PATENT APPLICATION

(11) **EP 2 377 885 A1**
(43) Date of publication of application: **19.10.2011**
(21) Application number: 09833533.4
(22) Date of filing: 18.12.2009
(51) Int. Cl.: C07K 16/02, A23K 1/18, A23L 1/305, A61K 39/395, A61P 31/00, C07K 16/12

(54) **METHOD FOR PRODUCING COMPREHENSIVE ANTI-SURFACE ANTIBODY WHEREIN THE ANTIGEN USED IS A MICROORGANISM FIXED BY A PROTEIN CROSSLINKING AND FIXATION REAGENT**

(30) Priority: 19.12.2008 JP 2008324257
(71) Applicant: Kabushiki Kaisha Saiwai Medix, Tokyo 101-0024 (JP)
(72) Inventor: SHICHIRI, Masayoshi, Tokyo 174-0065 (JP)
(74) Representative: Denison, Christopher Marcus
(86) International application number: PCT/JP2009/071716
(87) International publication number: WO 2010/071237

(57) **Abstract**

A method for preparing comprehensive antibodies against whole proteins that are expressed on the surface of a microorganism and the comprehensive antibodies obtained by the method are provided. The method for preparing comprehensive antibodies against whole proteins that are expressed on the surface of a microorganism comprises:
(a) treating a microorganism with a protein crosslinking and immobilization reagent and thus immobilizing proteins expressed on the surface of the microorganism via intramolecular crosslinking;
(b) administering the microorganism as an immunogen immobilized using the protein crosslinking and immobilization reagent to an animal; and
(c) obtaining antibodies from the animal.

## Description

### Technical Field

The present invention relates to a method for preparing antibodies and a method for preparing comprehensive antibodies against whole proteins that are expressed on the surface of a microorganism.

### Background Art

Numerous proteins are expressed in microorganisms such as bacterial cells and viruses. Conventionally, attempts have been made to select specific antigen protein molecules thought to be useful from these proteins, prepare monoclonal antibodies for the most part, and then apply the antibodies to medical practice or the like. However, there is a limit concerning specific recognition of a cell itself as a target using an antibody that recognizes a single antigen molecule, which is expressed on the surface of a bacterium or the like.

Mainly antibiotics have been conventionally used for preventing or treating bacterial or viral infectious diseases and the like. Also, a vaccine prepared using an attenuated virus has been administered to cause production of an antibody *in vivo* against a virus, following which the antibody has been used for preventing or treating the relevant viral infectious disease. However, antibiotics are problematic in that an appropriate antibiotic must be selected depending on bacteria, and resistant microbes emerge. Also, the safety of a vaccine must be taken into consideration, and thus infectious diseases to which vaccines are applicable have been limited.

Moreover, the use of an antibody specific to a verotoxin obtained by immunizing a chicken with the cells of enterohemorrhagic *Escherichia coli* as an antigen to treat infectious *Escherichia coli* diseases has been reported (see patent document 1). However, an object of the method is to produce an antibody against a verotoxin to be secreted by enterohemorrhagic *Escherichia coli* while using a cell of a microorganism as an antigen. No comprehensive antibodies against whole protein molecules that are expressed on the bacterial surface have been obtained. Also, the thus obtained antibody is used for neutralizing secreted verotoxin, and *Escherichia coli* itself cannot be eliminated by the method.
Patent document 1 JP Patent Publication (Kokai) No. H10-298105 A (1998)

### Summary of the Invention

An object of the present invention is to provide a method for preparing comprehensive antibodies against whole proteins that are expressed on the surface of a microorganism and comprehensive antibodies obtained by the method.

The present inventors have found that antibodies can be prepared broadly and comprehensively against whole immobilized antigen protein molecules by immobilizing the surface of a cell of a microorganism such as a virus or a bacterium using formaldehyde and then immunizing an animal with the resultant as an antigen. An antibody composition comprising such comprehensive antibodies has high specificity and sensitivity that have not been possible to obtain by conventional antibody preparation methods. The present inventors have also found that the antibody composition specifically and strongly recognizes an individual bacterium itself and is effective for treatments such as antimicrobial treatments, and they have completed the present invention.

The present invention is as follows.
[1] A method for preparing comprehensive antibodies including antibodies against whole protein molecules that are expressed on the surface of a microorganism, comprising:
   (a) treating a microorganism with a protein crosslinking and immobilization reagent and thus immobilizing proteins expressed on the surface of the microorganism via intramolecular crosslinking;
   (b) administering the microorganism immobilized using the protein crosslinking and immobilization reagent as an immunogen to an animal; and
   (c) obtaining antibodies from the animal.
[2] The method for preparing comprehensive antibodies according to [1], wherein the protein crosslinking and immobilization reagent is selected from the group consisting of formaldehyde, paraformaldehyde, and glutaraldehyde.
[3] The method for preparing comprehensive antibodies according to [1], wherein the protein crosslinking and immobilization reagent is 1% (v/v) to 38% (v/v) formalin.
[4] The method for preparing comprehensive antibodies according to any one of [1] to [3], wherein immobilization using the protein crosslinking and immobilization reagent is carried out for 10 minutes to 48 hours.
[5] The method for preparing comprehensive antibodies according to any one of [1] to [3], wherein the animal to which the immunogen is administered is a chicken.
[6] The method for preparing comprehensive antibodies according [5], wherein the antibodies are obtained from a hen egg laid by a chicken to which the immunogen has been administered.
[7] Comprehensive antibodies including antibodies against whole protein molecules expressed on the surface of a microorganism, which are obtained by the method according to any one of [1] to [6].
[8] A preventive or therapeutic agent for a microbial infectious disease comprising comprehensive antibodies including antibodies against whole protein molecules that are expressed on the surface of a microorganism, which is obtained by the method according to any one of [1] to [6].
[9] A food, comprising the comprehensive antibodies obtained by the method according to [6] from a hen egg.
[10] A feedstuff comprising the comprehensive antibodies obtained by the method according to [6] from a hen egg.
[11] A composition for treating an aquatic animal infectious disease comprising the comprehensive antibodies obtained by the method according to [6] from a hen egg.
[12] The composition for treating an aquatic animal infectious disease according to [11], which is used for application to environmental water inhabited by aquatic animals.

This description includes the disclosure of the description and drawings of Japanese Patent Application No. 2008-324257, from which the present application claims priority.

### Brief Description of the Drawings

Fig. 1 shows an outline of an aquaculture pond wherein ayu (sweetfish; Plecoglossus altivelis altivelis) cold water disease prevention tests were conducted using comprehensive antibodies.
Fig. 2 shows the results of ayu cold water disease prevention test 1 (cold water disease test results of test No. 1 conducted using hen egg antibodies in each aquaculture pond) using comprehensive antibodies.
Fig. 3 shows the results of ayu cold water disease prevention test 2 (cold water disease test results of test No. 2 conducted using hen egg antibodies in each aquaculture pond) using comprehensive antibodies.
Fig. 4 shows a photograph of ayu infected with cold water disease germs.
Fig. 5 is a photograph of ayu for which infection with cold water disease germs was avoided through administration of a feed comprising hen egg powder (hen egg antibodies) comprising comprehensive antibodies to the ayu.

### Embodiments for Carrying Out the Invention

Hereinafter, the present invention will be described in detail.

Antibodies can be prepared comprehensively against a microorganism by the method of the present invention. Here, the expression "..antibodies can be (are) prepared comprehensively against a microorganism" refers to simultaneous preparation of antibodies against whole numerous antigen protein molecules that are expressed on the surface of a microorganism. The thus obtained antibodies are referred to as comprehensive anti-surface antibodies. Comprehensive anti-surface antibodies are polyclonal antibodies including individual antibodies against numerous antigen proteins that are expressed on the surface of a microorganism. According to the method of the present invention, comprehensive anti-surface antibodies including antibodies against whole surface protein molecules can be obtained only by administration of an immobilized microorganism to an animal. Here, such antibodies against whole surface protein molecules are not required to include antibodies against all proteins expressed on the surface of a microorganism, but desirably include antibodies against most of surface proteins that exist in large quantities.

In the present invention, a microorganism for which comprehensive antibodies are prepared is immobilized using a reagent for crosslinking and immobilization within protein molecules. In this case, microbial bodies may be used as microorganisms such as cells. Examples of such a reagent for crosslinking and immobilization within protein molecules include aldehydes such as formaldehyde, paraformaldehyde, and glutaraldehyde. Preferably, immobilization is performed using formalin that is a 35%-38% and preferably 37% aqueous solution of formaldehyde.

When these reagents for crosslinking and immobilization within protein molecules are used as immobilization reagents, the reagents penetrate into microbial cells, intramolecular aldehyde groups bind to amino groups (α-amino group or ε-amino group of lysine residue) or SH groups of proteins on the surface of a microorganism, so as to form intermolecular crosslinking. Thus, proteins coagulate and are altered. Crosslinking deteriorates protein conformation, thereby inhibiting various biological activities such as enzymatic activity, transport, and secretion. An effect of the reagent for crosslinking and immobilization within protein molecules is a crosslinking reaction of protein molecules, so that no immobilization effect is exerted on a substance such as lipid and lipid and the like flow out.

When formalin that is a commercially available 35%-38% formaldehyde aqueous solution is used, immobilization may be performed using 1%-38% (v/v) formalin, preferably 2%-20% (v/v) formalin, and further preferably 5%-10% (v/v) formalin. Formalin to be used herein may be prepared using distilled water, saline, buffer, or the like.

Microorganisms such as cells are homogenized. Five (5) to 20 ml of formalin is added per gram of the thus homogenized pellets. Immobilization is performed by adding formalin to the homogenized microorganism, stirring the resultant, and then leaving the resultant to stand at 4°C-30°C for 30 minutes to 48 hours or more. Microorganisms that have survived are inactivated by short-time formalin treatment. The method of the present invention requires not only inactivation of microorganisms, but also formalin treatment to such an extent that protein molecules expressed on the surfaces of microorganisms form intramolecular crosslinking.

The thus immobilized homogenized microorganism is diluted with saline or buffer as necessary, suspended, and then mixed with a known adjuvant. Then the resultant may be administered as an immunogen to animals. Examples of animals to be used herein include mammals such as a mouse, a rat, nutria, a rabbit, sheep, a goat, a horse, and cattle, and birds such as a chicken and an ostrich. Particularly, birds such as chickens are preferable since hen eggs containing IgY antibodies can be obtained.

An animal can be immunized with an immunogen according to a known method. For example, immunization is performed by intraperitoneal or subcutaneous injection of an immunogen to an animal, as a general method. Immunization may be performed once or desirably performed via several times of administration every 2 to 10 days. Also, booster immunization may be performed several times every 5 to 10 days after the initial administration.

An example of a method for preparing bacterial immunogen is as follows. The following methods are examples, but the examples thereof are not limited to the following methods.

In the case of bacteria, they are collected by centrifugation and then pelletted. The pellet is added to formalin, dispersion is performed by stirring, and then the resultant is left to stand for 30 minutes. Subsequently, filtration is performed using rough filter paper and then the residue is removed. The filtrate is collected and then subjected to centrifugation. Phosphate buffer is added to the pellet and then the resultant is used as an immunogen.

The comprehensive anti-surface antibody composition of the present invention can be obtained from the serum of an immunized animal. Also, when an animal to be immunized is a chicken, the comprehensive anti-surface antibody composition can be obtained from hen eggs laid by the chicken. Within about 3 months after final immunization, hen eggs containing antibodies with high antibody titers can be obtained. Egg yolk of such a hen egg contains IgY and egg white contains IgA and IgM. When antibodies are collected from whole egg, the comprehensive antibody composition contains IgY, IgA, and IgM. When antibodies are collected from egg yolk, the comprehensive antibody composition contains IgY. When the antibody composition of the present invention is prepared using hen eggs, whole egg may be used or egg yolk alone may also be used.

The thus obtained hen eggs are broken and then egg yolk and egg white are stirred together or egg yolk alone is stirred, followed by powderization. At this time, antibodies are proteins and thus are sensitive to heat, so that they lose their activity at 70°C or higher. Powderization is performed at preferably 60°C or less. Preferably, drying is performed at 60°C or less by freeze-drying, spray drying, or hot air drying for powderization. The thus powdered hen eggs are further subjected to a pulverizer as necessary, thereby producing fine powders. The powders obtained by the method contain an oil component of egg yolk and thus have moisture. Such a fat content may be eliminated via complete delipidization using an ultracold critical method.

The comprehensive anti-surface antibody composition obtained from hen eggs may also be referred to as a hen egg antibody.

The antibody titers of the thus obtained antibodies can be measured by ELISA (enzyme-linked immuno adsorbent assay), EIA (enzyme immunoassay), RIA (radioimmunoassay), a fluorescent antibody technique, or the like. Antibody titer may be measured using the serum collected from an animal. Furthermore, when hen eggs are used, antibodies are extracted from hen eggs and then the antibody titers in the extracts are measured.

The comprehensive antibodies against a microorganism of the present invention can be used for preventing or treating microbial infectious diseases. The comprehensive antibodies of the present invention include antibodies against whole protein molecules that are expressed on the surface of a microorganism such as bacteria or viruses. Thus, the comprehensive antibodies have strong offensive power against a microorganism such as bacteria or viruses and can kill, eliminate, and control the microorganism such as bacteria or viruses.

Examples of such a microorganism include bacteria, viruses, fungi, rickettsiae, and parasites such as ciliates, which are pathogenic microorganisms that preferably infect animals and are pathogenic to animals.

Examples of such a microorganism are as follows, but the following microorganisms are given only for illustrative purposes and the examples are not limited thereto.

### Microorganisms that infect aquatic animals

Examples of an aquatic animal include fish, crustaceans, and mollusks. Examples of fish include cultured fish and ornamental fish, such as ayu (sweetfish; Plecoglossus altivelis altivelis), eel, trout, salmon, tuna, yellow tail, red sea bream, and flounder, aquarium fish such as goldfish, carp, and tropical fish. Examples of crustaceans include crabs and shrimps. Examples of mollusks include shellfish.

Examples of such a microorganism include an ayu cold water disease germ (*Flavobacterium psychrophilum*) that is a pathogenic bacterium of ayu cold water disease, *Flexibacter columnaris* that is a pathogenic bacterium of tail rot diseases, *Aeromonas salmonicida* that is a pathogen of hole diseases, *Aeromonas hydrophila* that is a pathogenic bacterium of aeromonas infectious diseases, *mizukabi* (fungi- or molds-like organisms that live in water) that is a fungal pathogen of wed fungal diseases, ciliates such as *Ichfhyophfhirius multifiliis* and *Cryptocaryon irritans* that are pathogenic parasites of white spot diseases, and carp herpes virus (KHV) and the like that are pathogenic viruses of carp herpes virus diseases. Other examples of such a microorganism include those pathogenic to fish, such as *Edwardsiella tarda, Enterococcus seriolicida, Flavobacterium branchiophilum, Flexibacter maritimus, Listonella anguillarum, Mycobacterium marinum, Nocardia seriolae, Pasteurella piscicida, Pseudomonas anguilliseptica, Pseudomonas fluorescens, Pseudomonas putida, Renibacterium salmoninarum, Staphylococcus epidermidis, Staphylococcus iniae,* and microorganisms of the genus *Vibrio.*

### Microorganisms that infect other animals

Examples of other animals include amphibians, reptiles, birds, and mammals.

Examples of such a microorganism include Gram-negative bacteria and Gram-positive bacteria. Examples of Gram-negative bacteria include *Pseudomonas aeruginosa, Vibrio cholerae,* and pathogenic *Escherichia coli.* Examples of Gram-positive bacteria include *Staphylococcus aureus* and the like. Further examples of the same include Trichophyton that is a fungal pathogen of tinea pedis, onychomycosis, or the like, *Pseudomonas aeruginosa* that causes sinusitis and the like, and pathogenic *Escherichia coli,* bacteria of the genus *Salmonella,* bacteria of the genus *Clostridium,* and bacteria of the genus *Campylobacter* that are pathogenic bacteria of intestinal infectious diseases. Other examples of the same include bacteria of the genus *Welchii* that are pathogenic bacteria of food poisoning, and bacteria of the genus *Serratia,* bacteria of the genus *Enterobacter,* and bacteria of the genus *Alcaligenes* that cause opportunistic infectious diseases.

Also, examples of viruses include influenza virus, HIV, poxvirus, herpes virus, adenovirus, parvovirus, baculovirus, picornavirus, togavirus, retrovirus, orthomyxovirus, paramyxovirus, rhabdovirus, reovirus, coronavirus, SARS coronavirus, tobacco mosaic virus, potyvirus, comovirus, and caulimovirus.

The antibody composition for preventing or treating microbial infectious diseases is used via administration to animals, for example. The antibody composition may be administered via oral route, nasal route, and intravenous, intramuscular, subcutaneous, and intraperitoneal injection or nonenteral route. For treatment of inflammation of nasal cavity or the like, the antibody composition can be administered in a liquid form or an aerosol form prepared by mixing with a known penetrating agent or in the form of paste in some cases. Also, for treatment of skin infections or the like, the antibody composition can be locally administered via application thereof in the form of liniment such as paste to an affected skin part or spraying of solutions to the same. The paste contains as a carrier, fat, fatty oil, lanolin, vaseline, paraffin, plastibase, wax, plaster, resin, plastic, glucols, higher alcohol, glycerin, water or emulsifier, or a suspending agent. The dose of the pharmaceutical composition of the present invention differs depending on symptom, age, body weight, and the like. Generally in the case of oral administration, the dose may range from about 0.01 mg to 10000 mg per day for an adult and can be administered once or several separate times.

The antibody composition for preventing or treating microbial infectious diseases may contain a carrier, a diluent, and an excipient that are generally used in the field of drug formulation. For example, as a carrier for tablets and an excipient, lactose, magnesium stearate, and the like are used.

Moreover, a microbial infectious disease of an animal can be prevented or treated by mixing the comprehensive antibodies against a microorganism of the present invention to a feedstuff for the animal and then administering the feedstuff to the animal.

Also, in the case of infectious diseases of aquatic animals, the antibody composition against a microorganism of the present invention may be added into environmental water (water for breeding) in aquariums, aquaculture ponds, preserves for aquaculture, and the like inhabited by aquatic animals. In this case, such a therapeutic antibody is incorporated into the bodies of aquatic animals via oral route so as to be able to exert the effects *in vivo.* Moreover, the therapeutic antibody binds to the microorganism existing in environmental water, so as to inactivate the microorganism. Also, the therapeutic antibody binds to the microorganism, so as to form water-insoluble aggregates of the antibody and the microorganism, the aggregates are then precipitated, and thus the aggregates are eliminated from a drain ditch of the aquaculture pond, for example, and specifically the aggregates are removed from the environmental water.

Specific examples of therapeutic methods for infectious diseases of cultured fish or crustaceans are as described below. Examples of infectious diseases of cultured fish include, but are not limited to, ayu cold water disease and the like caused by Flavobacterium (cold water disease germ) as a causative bacterium, and hole disease caused by Pseudomonas as a causative bacterium. For prevention or treatment of infectious diseases of fish, proteins that are expressed on the surfaces of the cells of causative bacteria are immobilized, an immunogen is prepared, chickens are immunized with the immunogen, and thus a hen egg antibody is obtained. The thus obtained hen egg antibody is mixed with a fish feed and then the fish feed is administered via feeding. Also, the thus obtained hen egg antibody may be added to environmental water inhabited by cultured fish.

Hen egg antibodies are administered together with the feed via feeding, so that the hen egg antibodies are incorporated into the digestive tracts of fish bodies and some of the antibodies are dissolved or suspended in water for breeding (in which fish are bred) without being incorporated into fish bodies. The antibodies dissolved in water for breeding react with bacteria suspended in the water for breeding to bind to cells and thus kill the microbial cells.

The present invention encompasses foods or feedstuffs comprising comprehensive antibodies obtained from hen eggs. Furthermore, the present invention encompasses a composition for treating infectious diseases of aquatic animals, comprising the comprehensive antibodies obtained from hen eggs. The composition for treating infectious diseases of aquatic animals may be supplied as a feed to aquatic animals or added into environmental water for application.

The present invention is further specifically illustrated with reference to the following examples. However, these examples do not limit the present invention. Example 1 Preparation of comprehensive antibody using bacterium as antigen

As an antigen, cells of ayu cold water disease germ were used.

The ayu cold water disease germ (*Flavobacterium psychrophilum*) was cultured in Cytophaga medium (or TYES medium [triptone 0.04%, yeast extract 0.005%, MgSO₄, 7 H₂O 0.005%, CaCl₂/2H₂0 0.005%, pH 7.2]) at 18°C and then cultured in agar plate medium at 18°C for 5 days. Thus, after confirmation of the formation of yellow colonies having irregular wave-like edges (photograph), the culture solution was centrifuged to pellet the cells. The pellets were cryopreserved until the use as antigens.

About 3 mg of the thawed pellets of the ayu cold water disease germ was added to a 15-ml capped test tube, 5 ml of 5% (v/v) formalin solution was added to the test tube, and then the test tube was sealed.

The solution was stirred several times (about 10 minutes) using a test tube mixer. The pellets were completely dissolved in a 5% (v/v) formalin solution (Junsei Yakuhin: the formalin solution containing 35.0%-38.0% formaldehyde and 5%-13% methanol was diluted with water) until they were diffused and dissolved uniformly. Incubation was carried out for 15 minutes, so as to immobilize the surface antigens of *Staphylococcus aureus.* After completion of incubation, the resultant was left to stand for 30 minutes.

The solution was filtered through rough filter paper moistend in advance with a small amount of distilled water for injection, and then the residue was removed. At this time, natural filtration was carried out using gravity when a preparation amount was low, and suction filtration was carried out when a preparation amount was high. A filtrate containing bacterial cells was recovered and then centrifuged at a high speed (rotation frequency; 15000 rpm or more for 30 minutes or more).

The supernatant was discarded and then about 1.5 ml of phosphate buffer was added to about 3 mg of pellets, so that the pellets were dispersed. The resultant was used as an antigen to be administered.

The antigen composition to be administered was prepared by gradually mixing 1 ml of the antigen and 1 ml of white oil (Nippon Shinyaku Co., Ltd.) with an emulsion pump so as to mix them and thus prepare an emulsion.

The thus prepared antigen composition emulsion (0.5 ml) was subcutaneously administered to the inguinal region of each 5-week-old female chicken. At this time, the antigen composition to be administered was heated in advance to chicken body temperature, 37°C, in order to lower the invasiveness to the chicken.

When formalin separation by centrifugation after immobilization was insufficient, some chickens huddled because of pain and showed no feeding behavior, except for drinking water for about 3 days after administration of the antigen. Some chickens died during the period. The site to which the antigen had been administered became hardened, exhibiting a state of induration.

Every 5 days after the initial administration, the antigen composition was administered twice for booster immunization. Thus, a total of 3 instances of immunization were carried out. Antibodies could be obtained from hen eggs laid by immunized chickens. Within about 3 months after the final immunization, hen eggs containing antibodies with high antibody titers could be obtained. Dry hen egg powder was produced using a spray dryer (GA22 type, Yamato Scientific Co., Ltd.), a hot air dryer, and a pulverizer.

### Example 2 Prevention of ayu cold water disease using comprehensive anti-surface antibody

The hen egg powder prepared in Example 1 containing the antibodies against the ayu cold water disease germ was used. Here, the "hen egg powder containing the antibodies" is also referred to as "hen egg antibodies."

Stream aquaculture ponds for ayu (sweetfish; Plecoglossus altivelis altivelis) in 3 areas (Area 1: Nagahama-shi, Shiga-ken, Japan; Area 2: Shiga-machi, Shiga-ken, Japan; and Area 3: Chinai-gawa river system, Shiga-ken, Japan) were used.

About-100-m² aquaculture ponds were used. Two aquaculture ponds were used in Area 1 and Area 2, and one aquaculture pond was used in Area 3. Also, in each area, an about-100-m² control aquaculture pond was prepared, in which ayu were bred via administration of a conventionally employed fishery medicine for prevention of ayu cold water disease, Isran soda (general name: sulfisozolesodium), and novobiocin, a fishery medicine for prevention of vibrio diseases without supplying any hen egg antibody.

Tests were conducted for over 1 year and 3 months, during which a 4-month measurement period was provided twice, once for Test 1 and once for Test 2.

For the tests, young fish born in lake Biwa, Shiga-ken, Japan and captured by trap fishery (using a set net referred to as an "eri"), were used, but marine ayu and hatchery-reared ayu were not used.

The number of ayu in the aquaculture ponds ranged from 532300 to 833300 (with total weight ranging from 315 kg to 450 kg) in Test 1 and the same ranged from 603773 to 882352 (total weight ranging from 320 kg to 450 kg) in Test 2.

Hen egg antibody powder was mixed at a 3:100 (3%) ratio by weight with respect to the feed to be administered. In each aquaculture pond, a feed containing the hen egg antibody was administered at a 2:100 (2%) to 5:100 (5%) ratio by weight with respect to the total fish body weight (= total weight of fish bodies). Feeding was carried out 5 to 6 times a day. Total feed amounts were not unified because of difficulty in unifying the amounts.

In the aquaculture ponds, during the tests, conventionally performed methods for prevention of ayu cold water disease, such as administration of antibiotics and hot water treatment, were not applied.

During the test periods, dead fish were collected at 5 p.m. every day, and the causes of death (death by disease or death by natural causes) were determined. For fish that had died from disease, it was further determined whether they had died from cold water disease or another disease (i.e., death by bacterial infectious diseases due to bacteria such as those of Aeromonas or Pseudomonas, or death by parasites). Cold water disease can be determined on the basis of symptoms such as poverty of blood in the branchial mantle or internal organs, bleeding in the lower part of the branchial mantle, fading of coloration on the body surface, perforation on the body surface due to ulcer formation on the body surface, anal dilatation, bladder enlargement, mandibular defects, and tail fin defects.

The hen egg antibodies were directly administered together with the feed, so that they were incorporated into the digestive tracts of fish bodies, but some of the hen egg antibodies were dissolved into pond water without being incorporated into fish bodies. The antibodies dissolved in pond water then reacted with cold water disease germs suspended in the pond water, so as to become bound to cells. Also, the antibodies dissolved in pond water were incorporated into the branchial mantle upon gill respiration by ayu. Thus, the antibodies became strongly bound to the surface antigens of cold water disease germs existing within the branchial mantle, existing on the fish body surfaces, or infecting the lesions of ayu, thereby achieving improvement of symptoms. All cold water disease germs existing in such a manner in water were targeted by and reacted with the antibodies. Cells to which the antibodies had been bound were precipitated in the bottom of each pond and discharged via the central drain ditch to the outside of the aquaculture pond.

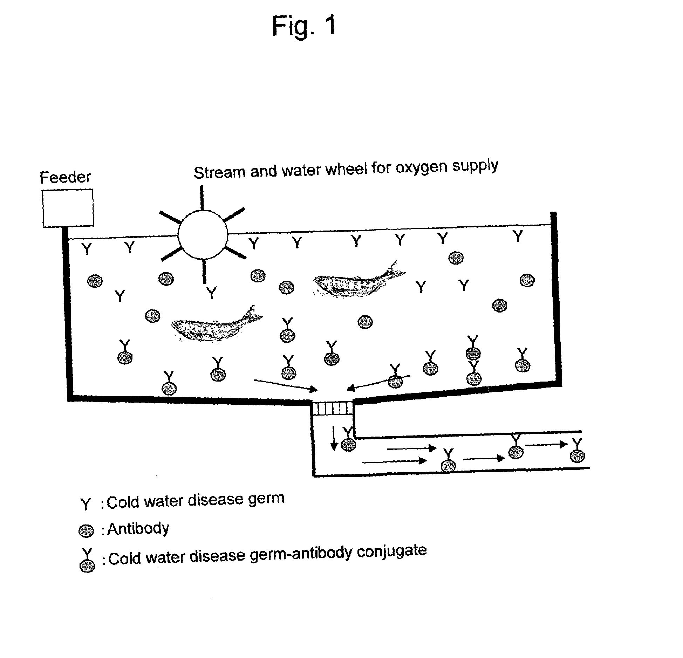

Fig. 1 shows an outline of an aquaculture pond. Fig. 1 shows the antibodies, cold water disease germs, and how the antibodies and cold water disease germs exist.

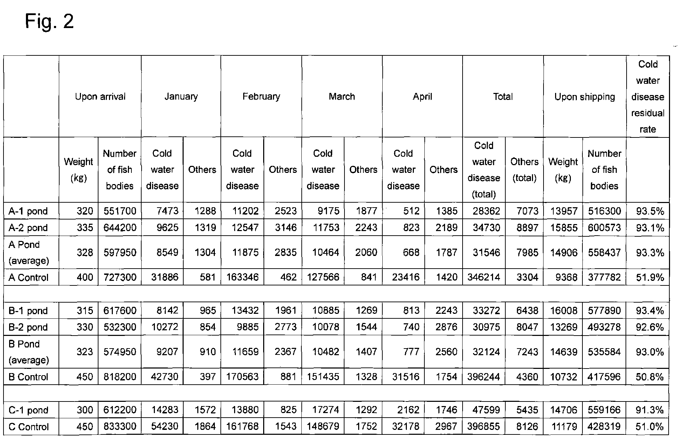

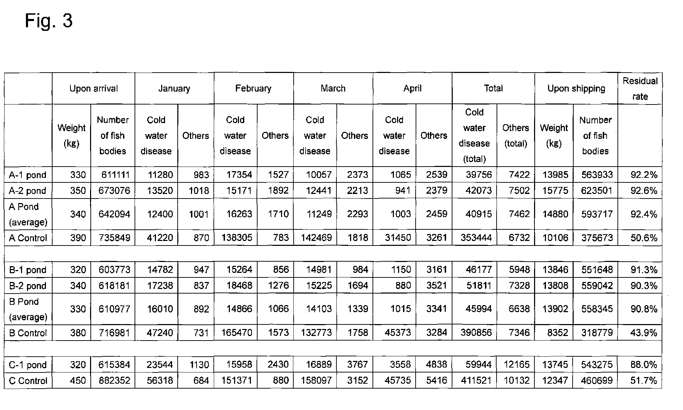

Fig. 2 shows the results of Test 1. Fig. 3 shows the results of Test 2.

In Fig. 2 and Fig. 3, the aquaculture ponds in the 3 areas are pond A-1 and pond A-2, B-1 pond and pond B-2, and pond C-1, respectively; and the aquaculture ponds used as control ponds in the 3 areas are referred to as A control (AC), B control (BC), and C control (CC), respectively.

Both Test 1 and Test 2 were conducted for 4 months. Fig. 2 and Fig. 3 show the numbers of fish that died on a monthly basis from cold water disease and the same figures for fish that died from other causes. Aquaculture was initiated in late December of the previous year.

In Fig. 2, the residual percentage was calculated by dividing the total weight of young fish introduced into each pond by the average weight of 10 fish and dividing the total weight of the fish at the time of shipping by the average weight of 10 fish.

In Test 1, the average fish body weight of 100 fish in January was 0.58 g in pond A-1, 0.55 g in pond A-2, 0.57 g in AC, 0.51 g in pond B-1, 0.59 g in pond B-2, 0.55 g in BC, 0.49 g in pond C-1, or 0.54 g in CC. The average fish body weight of 100 fish at the end of April was 25.4 g in pond A-1, 26.4 g in pond A-2, 24.8 g in AC, 27.7 g in pond B-1, 26.9 g in pond B-2, 25.7 g in BC, 26.3 g in pond C-1, and 26.1 g in CC.

In Test 2, the average fish body weight of 100 fish in January was 0.54 g in pond A-1, 0.52 g in pond A-2, 0.53 g in AC, 0.53 g in pond B-1, 0.55 g in pond B-2, 0.53 g in BC, 0.52 g in pond C-1, and 0.51 g in CC. The average fish body weight of 100 fish at the end of April was 24.8 g in pond A-1, 25.3 g in pond A-2, 26.9 g in AC, 25.1 g in pond B-1, 24.7 g in pond B-2, 26.2 g in BC, 25.3 g in pond C-1, and 26.8 g in CC.

As shown in Fig. 2 and Fig. 3, in all of the 3 areas, the residual percentage was significantly increased in ponds in which the feed containing the hen egg antibody had been administered, compared with the cases of the control ponds.

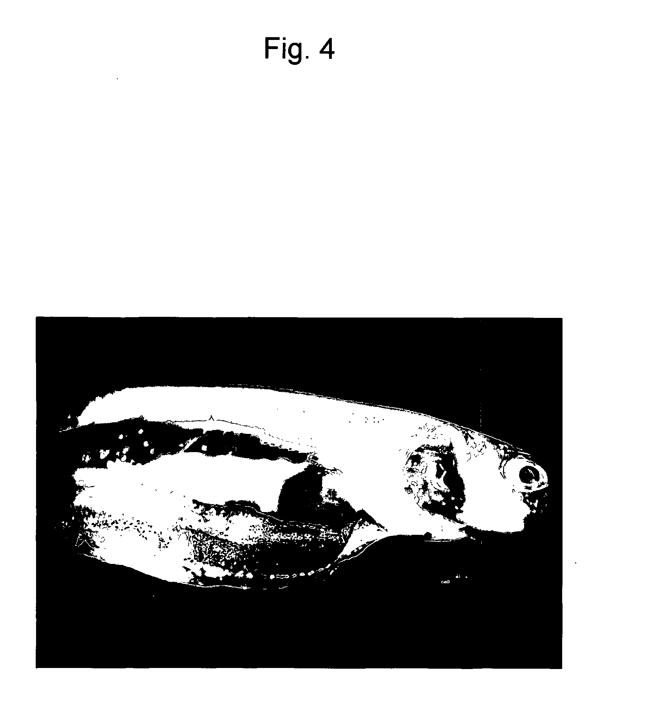

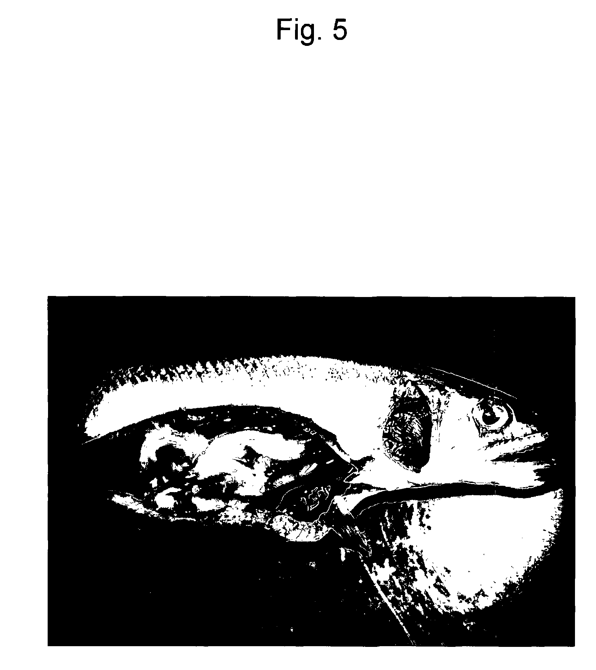

Fig. 4 shows a photograph of ayu infected with cold water disease germs. In the ayu infected with cold water disease germs, internal organs were dissolved and the gill and the heart became white because of poverty of blood. Fig. 5 shows a photograph of ayu that had been administered with the feed containing the hen egg antibody. Both color and morphology of the internal organs were normal, and the gills were filled with blood with high oxygen saturation, presenting red coloration.

### Industrial Application

According to the method of the present invention, proteins expressed on the surface of a microorganism are immobilized via intramolecular crosslinking using a reagent for crosslinking within protein molecules, and then the resultants are administered to an animal, so that comprehensive antibodies including antibodies against whole proteins expressed on the surface of a microorganism can be obtained.

The comprehensive antibodies bind to the entire surface of a microorganism, and thus they are able to inactivate or kill the microorganism. In particular, the comprehensive antibodies can be used for preventing or treating microbial infectious diseases.

All publications, patents, and patent applications cited herein are incorporated herein by reference in their entirety.

## Claims

1. A method for preparing comprehensive antibodies including antibodies against whole protein molecules that are expressed on the surface of a microorganism, comprising:
(a) treating a microorganism with a protein crosslinking and immobilization reagent and thus immobilizing proteins expressed on the surface of the microorganism via intramolecular crosslinking;
(b) administering the microorganism immobilized using the protein crosslinking and immobilization reagent as an immunogen to an animal; and
(c) obtaining antibodies from the animal.

2. The method for preparing comprehensive antibodies according to claim 1, wherein the protein crosslinking and immobilization reagent is selected from the group consisting of formaldehyde, paraformaldehyde, and glutaraldehyde.

3. The method for preparing comprehensive antibodies according to claim 1, wherein the protein crosslinking and immobilization reagent is 1% (v/v) to 38% (v/v) formalin.

4. The method for preparing comprehensive antibodies according to any one of claims 1 to 3, wherein immobilization using the protein crosslinking and immobilization reagent is carried out for 10 minutes to 48 hours.

5. The method for preparing comprehensive antibodies according to any one of claims 1 to 4, wherein the animal to which the immunogen is administered is a chicken.

6. The method for preparing comprehensive antibodies according claim 5, wherein the antibodies are obtained from a hen egg laid by a chicken to which the immunogen has been administered.

7. Comprehensive antibodies including antibodies against whole protein molecules expressed on the surface of a microorganism, which are obtained by the method according to any one of claims 1 to 6.

8. A preventive or therapeutic agent for a microbial infectious disease comprising comprehensive antibodies including antibodies against whole protein molecules that are expressed on the surface of a microorganism, which is obtained by the method according to any one of claims 1 to 6.

9. A food, comprising the comprehensive antibodies obtained by the method according to claim 6 from a hen egg.

10. A feedstuff comprising the comprehensive antibodies obtained by the method according to claim 6 from a hen egg.

11. A composition for treating an aquatic animal infectious disease comprising the comprehensive antibodies obtained by the method according to claim 6 from a hen egg.

12. The composition for treating an aquatic animal infectious disease according to claim 11, which is used for application to environmental water inhabited by aquatic animals.
